# EUROPEAN PATENT SPECIFICATION

(11) **EP 2 291 223 B1**
(45) Date of publication and mention of the grant of the patent: **06.01.2016**
(21) Application number: 09730484.4
(22) Date of filing: 09.04.2009
(51) Int. Cl.: A61N 1/40

(54) **DEVICE FOR RADIOFREQUENCY CIRCULAR DEEP THERAPY**
VORRICHTUNG ZUR KREISFÖRMIGEN TIEFEN RADIOFREQUENZ-THERAPIE
APPAREIL DE THÉRAPIE PROFONDE CIRCULAIRE PAR RADIOFRÉQUENCE

(30) Priority: 11.04.2008 SI 200800087
(43) Date of publication of application: 09.03.2011
(73) Proprietor: Iskra Medical, D.O.O., 4244 Podnart (SI)
(72) Inventor: JELENC, Jure, 4244 Podnart (SI); JELENC, Joze, 4244 Podnart (SI)
(74) Representative: Zacco GmbH
(86) International application number: PCT/SI2009/000014
(87) International publication number: WO 2009/126117

(56) References cited:
- WO-A-2005/112807
- WO-A-2007/093998
- WO-A-2007/096009
- JP-A- 3 162 870
- US-A1- 2006 036 300
- US-A1- 2007 088 413
- US-B1- 6 500 141

## Description

### Field of Invention

The present invention relates to a device for radiofrequency (RF) circular deep therapy allowing an efficient performance of deep therapy by means of radiofrequency heating of subcutaneous tissue.

### Technical Problem

A technical problem is a constructional solution to such device that would allow performance of therapy with two electrodes in the same application handpiece on the principle of capacitive radiofrequency by an optimum distribution of the RF electric field. A problem in the construction of two capacitor electrodes is optimisation of size, shape and especially areal arrangement of capacitor electrodes in a device handpiece, since the arrangement of electrodes has an essential influence on the quality of therapy in bipolar RF methods. The goal is to design capacitor electrodes in a way that would provide for such arrangement of electrodes in an area, in which mutual distance and the surface of electrodes would allow for a maximum yield of the RF source. The shape and superficial ratios of electrode sizes of the capacitor must allow the best possible contact with patient's tissue and the most possible even distribution of the electric field over the surface of patient's skin by excluding local maximums of electric fields. A constructional solution to a device's handpiece should offer a possibility of simultaneous performance of vacuum therapy.

### Prior Art

Radiofrequency therapy is used in physiotherapy, aesthetic surgery, dermatology and cosmetics. It may be applied in all fields where the heating of tissues is needed. Therapy based on high-frequency induced voltage having a frequency ranging from 0.1 MH up to a few 10 MH, preferably from 0.4 MH to 6 MH, causes heating in the body that is desired as useful to be achieved in subcutaneous tissues. Basically, there are two principles of operation: resistive - based on ohmic resistance, and capacitive - based on capacitive resistance.

During the operation of a device for therapy based on the resistive method, ohmic currents appear on patient's skin after the voltage in the conductor, i.e. a body or patient's skin between two electrodes is switched on. The current flows through the body from a first, smaller electrode, to a second, larger one. Since the current is the same through both electrodes, the size of the electrode has a strong influence on the density of electric currents. The smaller electrode causes the desired heating in the body, whereas the larger one has a lower density of electric current thus causing no effect. Unfortunately, due to high ohmic resistance of the skin towards the subcutaneous tissue a considerable decrease in voltage appears thus causing the heating in epidermis and upper skin layers leading to undesired burns. This process is even more obvious when the contact surface of the electrode gets undesirably decreased due to various reasons if the electrode fails to completely fit. When these reasons appear they are accompanied by an extremely unpleasant, burning feeling. Some manufacturers of such devices try to overcome this problem by various ways of skin cooling. Better feelings during therapy are enabled by analgesia of the skin upon its cooling. A drawback of such therapy lies in the fact that a patient does not feel burns and is not aware of the necessity for the discontinuation of therapy when damage is caused.

US2007265614 discloses a solution to a method described above by an RF signal, which means that the applicator is not insulated. The solution is monopolar and disposes over a device for patient's skin cooling.

US20020072610 discloses a resistive solution similar to the one described above. This solution does away with the disadvantage linked to the shape of rectangular electrodes, in which huge electric fields appear on the edges thus causing an uneven distribution of therapy effects under the surface of the applicator. This is unfavourable since no even therapy can be achieved on the entire area and moreover the densities of the electric field on the edges become that huge to cause damages. The solution thus comprises a round applicator having no edges, which contributes to a more equal distribution of the field on the surface and no local maximums appear.

Methods using the capacitive method are known in existing solutions. Electrodes in capacitive radiofrequency are insulated by a non-conductive substance / insulator. Only capacitive current not causing heating runs between the electrodes being capacitor panels. Its high frequency causes potent polarisation of dipolar water molecules in tissue. These try to follow the frequency of the forced voltage of the electric field in which they are present and consequently cause the heating of tissue. Heating is caused in tissues consisting of polarised dipoles (water), in lower skin layers and fat tissues. It does not have influence on the upper skin layers, since it does not contain a sufficient quantity of dipolar molecules (water) for more considerable heating. This ensures heating in subcutaneous tissues.

Known radiofrequency devices with a capacitive mode of operation function on the principle of a small active electrode and a larger remote one. The density of capacitive currents on the larger remote electrode is small enough not to cause effects on tissue. This is a monopolar mode. The bipolar mode presents a use of two electrodes within the same applicator. The used electrodes are of similar size and therefore exert the same influence on tissue. An advantage of this method is that the applied RF voltage is considerably lower due to two-fold operation.

US2007255274 discloses a solution based on a capacitive method that may have one or several electrodes and can function in a monopolar or bipolar mode. The solution is based on one or several first electrodes and on only one second electrode of the capacitor. The electrodes are arranged one next to another, which decreases the homogeneity of the RF field. Transmitting electrodes are mutually coupled, which calls for a very technologically demanding solution.

The US20070088413 application discloses a capacitive bipolar method having two or more electrodes arranged one next to another, which decreases the homogeneity of the field.

The Slovene patent No. 20906 discloses a device for circular deep skin therapy characterised by vacuum therapy by means of massage balls.

The Lumenis company and a few other manufacturers use a combination of resistive RF and vacuum therapy. The used method is a resistive method having the drawbacks as described above.

### Solution to the Technical Problem

The essence of a device for radiofrequency circular deep therapy lies in that two electrically insulated electrodes of a capacitor are housed in the handpiece of a device. The first circular electrode is arranged on the bottom of a cylindrical support encircled by a bell-shaped support with a collar housing a second electrode in the shape of a circular ring. The bell-shaped support creates the conditions for vacuum therapy.

The device for radiofrequency circular deep therapy of the invention will now be described by means of drawings, on which show:
Figure 1 - handpiece of the device for radiofrequency circular deep therapy in cross-section
Figure 2 - plan view of the handpiece of the device for radiofrequency circular deep therapy
Figure 3 - handpiece of the device for radiofrequency circular deep therapy in cross-section

The device for radiofrequency circular deep skin therapy of the invention disposes apart from a typical handpiece over a power supply, a generator of a radiofrequency signal and a vacuum pump. The handpiece of the device houses two electrically insulated electrodes of a capacitor arranged in a way that the first electrode of the capacitor of a circular shape is encircled by another electrode in the shape of a circular ring. The device may also be without the vacuum pump.

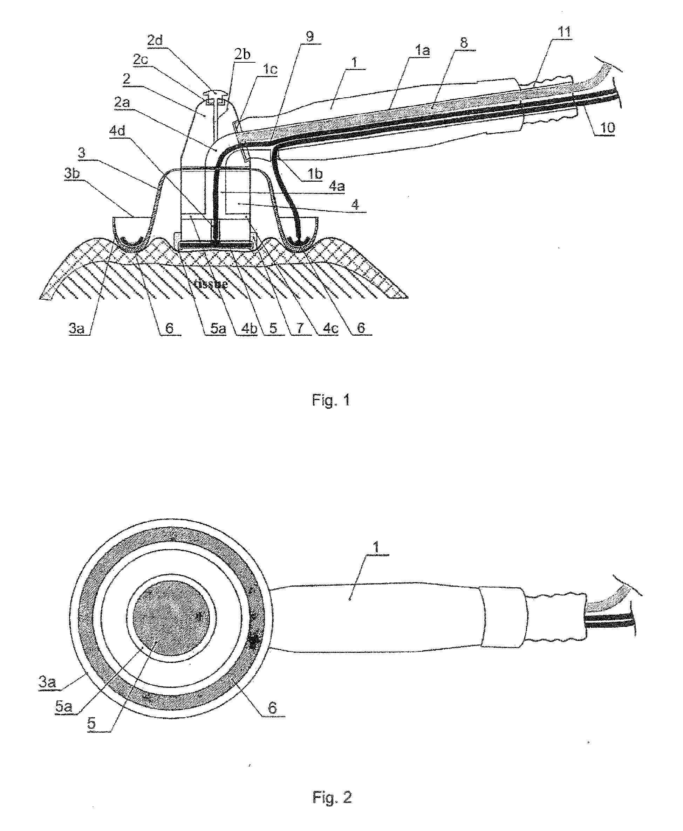

The handpiece of the device consists of a grip 1, a support 2, a bell-shaped support 3 with an electrode 6 and a cylindrical support 4 with a round electrode 5. As evident from Figure 1, the ergonomically shaped grip 1 has a groove 1a in the centre along its length that terminates in a short cylindrical groove 1 b in the vicinity of said support 2. Said grip 1 has threaded threads on a part 1c intended for the screwing into said support 2. Said support 2 is cupola-shaped and has in its interior a quarter-circularly shaped groove 2a and a narrow groove 2b terminating in a drilled hole 2c, in which a cover 2d is positioned. During the operation of the device with vacuum said cover 2d is in close contact with said support 2. The vacuum suctioned handpiece is relieved by lifting said cover 2d, said groove 2b is opened and the ambient air enters said bell-shaped support 3. The lower part of said support 2 is provided with threads, which serve to screw said bell-like support 3, after having been positioned, into said cylindrical support 4. Said cylindrical support 4 has a wide vertical groove 4a in the centre and horizontal grooves 4b and 4c. A vertical groove 4d runs from the lower line of grooves 4b and 4c in the centre of the support 4. A round electrode 5 of the capacitor is fastened together with a dielectric housing 5a to the bottom of said support 4 by means of a member 7.

Said bell-shaped support 3 is provided with a hole in the centre intended for fastening into an integral part with said supports 2 and 4. From the horizontal part of said support 3 the side equally extends downwards to approximately 1/6 of the height and forms a semicircular collar 3a, into which said ring-shaped electrode 6 of the capacitor is arranged. Said electrode 6 is bent in a way to adapt to the curve of said collar 3a in the part of said support 3 closest to patient's skin. Said collar 3a is closed by said ring-shaped cover 3b.

An air suction tube 8, a wire 9 for the supply of said electrode 5 and a wire 10 for the supply of said electrode 6 are arranged through said groove 1a of said handpiece 1. Said wire 9 is also introduced through said groove 2a of said support 2 and 4d of said support 4. At the other end of said handpiece 1 there is a flexible tube 11 leading to the housing of said device and allows for safe handling of said device.

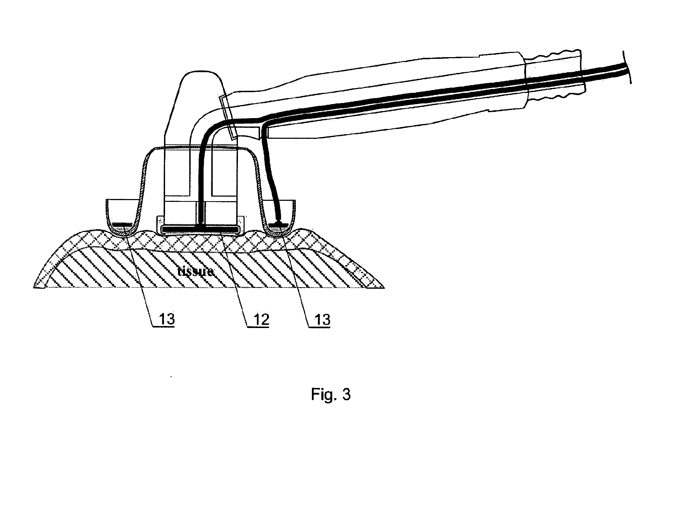

In compliance with Embodiment I of the device of the invention intended for treatment without vacuum, an electrode 13 is provided in the shape of a horizontal circular ring around said round electrode 12 as shown in Figure 3.

The device of the invention with vacuum can dispose over a further separate third electrode. A variant without a handpiece is also possible. In this variant the first and second electrodes are connected to an electrical contact in a way that they represent one electrode, wherein another, neutral electrode in the shape of a rectangular or round plate is arranged on the remote part of the body and connected to a generator of RF signal as well.

There is a possibility to include one or several ultrasound sources above the central electrode acting on the tissue on a mechanical and/or thermal basis. The source may be made of a piezoelectric crystal.

In order to achieve additional therapeutic effects on tissue, one or several sources of visible and/or infrared light may be inserted in the handpiece of the device of the invention.

Two, three or even more mutually separated electrodes of a capacitor may be inserted into the handpiece of the device of the invention, said electrodes being positioned one into another. To achieve special distributions of RF fields an oval shape of electrodes may be used instead of round ones.

The device of the invention is intended for applications in aesthetic medicine, cosmetics, physiotherapy, dermatology, oncology, veterinary medicine as well as in other fields where deep heating is useful. The effects of therapy with radiofrequency are based on tissue heating and among others on the activation of the autoimmune process of a body. A simultaneous use of vacuum therapy considerably contributes to better blood circulation of the treated area, which results in a better and more rapid effect of the immune response of the body. Simultaneous increased blood circulation improves the dielectric property of tissue thus increasing the efficiency of therapy. The round shape of said electrode 5 having said dielectric housing 5a and said support 3 provides for a smooth gliding over the surface and facilitates pleasant therapy. During the therapy with the device of the invention oils having good dielectric properties may be used in order to diminish friction and to improve smooth gliding of the handpiece of said device on the skin.

The device of the invention offers the best ratio between surface area and circumference provided for by the circular shape of the electrode. The greatest surface area possible of electrodes of the capacitor upon their simultaneous greatest remoteness possible is achievable through a positioning of a circular electrode into another electrode, which is also of a circular shape. With a round shape of electrodes the electric field reaches optimal homogeneity in all points. Great mutual distance between electrodes is the reason for increased depth of operation of radiofrequency currents and increased surface of tissue treated at a time. Moreover, it is important to reach a greater surface of active insulated electrode in contact with skin, because the yield is thus improved and the power of the radiofrequency generator needed decreased.

## Claims

1. Device for circular deep skin therapy by capacitive application of radiofrequency electric fields, said device comprising an application handpiece, a power supply, and a generator of a radiofrequency signal, the handpiece of the device housing a first (5, 12) and a second (6, 13) electrically insulated electrode of a capacitor, wherein said first electrode (5, 12) of the capacitor is of a circular shape, is housed within a dielectric housing (5a) and is arranged on the bottom of a cylindrical support (4) encircled by a bell-shaped support (3) having a collar (3a) housing said second electrode, said second electrode assuming the shape of a circular ring and encircling said first electrode.

2. Device for circular deep skin therapy as claimed in Claim 1, further comprising a vacuum pump.

3. Device for circular deep skin therapy as claimed in Claim 1, **characterised in that** said handpiece of said device consists of a grip (1), a support (2), **in that** said grip (1) is ergonomically shaped and has a groove (1a) in the centre along its length that terminates in a short cylindrical groove (1b) in the vicinity of said support (2), **in that** said grip (1) has threaded threads on a part (1c) intended for the screwing into said support (2), **in that** said support (2) is cupola-shaped and has in its interior a quarter-circularly shaped groove (2a) and a narrow groove (2b) terminating in a drilled hole (2c), in which a cover (2d) is positioned, **in that** during the operation of the device with vacuum said cover (2d) is in close contact with said support (2), **in that** the lower part of said support (2) is provided with threads serving to screw said bell-shaped support (3), after having been positioned, into said cylindrical support (4), **in that** said cylindrical support (4) has a wide vertical groove (4a) in the centre and horizontal grooves (4b and 4c), **in that** a vertical groove (4d) runs from the lower line of grooves (4b and 4c) in the centre of said support (4), **in that** said first electrode (5) of the capacitor is fastened together with said dielectric housing (5a) to the bottom of said support (4) by means of a member (7), **in that** said bell- shaped support (3) is provided with a hole in the centre intended for fastening into an integral part with said support (2) and cylindrical support (4), **in that** from the horizontal part of said bell-shaped support (3) the side equally extends downwards to approximately 1/6 of the height and forms a semicircular collar (3a), into which said ring-shaped electrode (6) of the capacitor is arranged, **in that** said electrode (6) is bent in a way to adapt to the curve of said collar (3a) in the part of said support (3) which is, in use, closest to patient's skin, **in that** said collar (3a) is closed by a ring-shaped cover (3b), **in that** an air suction tube (8), a wire (9) for the supply of said first electrode (5) and a wire (10) for the supply of said second electrode (6) are arranged through said groove (1a) of said handpiece (1), **in that** said wire (9) is also introduced through said circularly shaped groove (2a) of said support (2) and said vertical groove (4d) of said cylindrical support (4), **in that** at the other end of said handpiece (1) there is a flexible tube (11) leading to a housing of said device and allowing for safe handling of said device.

4. Device for circular deep skin therapy as claimed in Claim 1 or 2, **characterised in that** said second electrode (13) has a shape of a horizontal circular ring.

5. Device for circular deep skin therapy as claimed in Claim 1 or 2, **characterised in that** said second electrode (6) is bent in a way to adapt to the curve of said collar (3a) in the part of said support (3) which is, upon use, closest to the patient's skin.

6. Device for circular deep skin therapy as claimed in any one of Claims 1 to 5, **characterised in that** said electrodes (5, 6, 12, 13) are electrically coupled to form one electrode, wherein another, neutral electrode in the shape of a rectangular or round plate is adapted to be arranged on a remote part of the body and connected to the generator of RF signal

7. Device for circular deep skin therapy as claimed in Claims 1 to 6, **characterised in that** a further separated third electrode is integrated into said handpiece

8. Device for circular deep skin therapy as claimed in Claims 1 to 7, **characterised in that** one or several ultrasound sources are inserted above the first electrode acting on the tissue on a mechanical and/or thermal basis.

9. Device for circular deep skin therapy as claimed in Claims 1 to 7, **characterised in that** an ultrasound source is inserted above said first electrode made of a piezoelectric crystal.

10. Device for circular deep skin therapy as claimed in Claims 1 to 7, **characterised in that** one or several sources of visible and/or infrared light are inserted into said handpiece of the device.

11. Device for circular deep skin therapy as claimed in Claims 1 to 7, **characterised in that** two, three or even more mutually separated and one within another arranged electrodes of a capacitor are inserted into said handpiece of the device.

12. Device for circular deep skin therapy as claimed in Claims 1 to 11 , **characterised in that** said first electrode of the capacitor is of oval shape and encircled by said second electrode, which has a shape of an oval ring.

## Patentansprüche

1. Vorrichtung für kreisförmige Hauttiefenbehandlung durch kapazitive Anwendung von elektrischen Hochfrequenzfeldern, wobei die Vorrichtung ein Applikationshandstück, eine Stromversorgung und einen Erzeuger eines Hochfrequenzsignals umfasst und das Handstück der Vorrichtung eine erste (5, 12) und eine zweite (6, 13) elektrisch isolierte Elektrode eines Kondensators aufnimmt, wobei die erste Elektrode (5, 12) des Kondensators eine Kreisform aufweist, innerhalb eines dielektrischen Gehäuses (5a) aufgenommen ist und auf der Unterseite eines zylindrischen Trägers (4) angeordnet ist, der von einem glockenförmigen Träger (3) umgeben ist, welcher einen Kragen (3a) aufweist, der die zweite Elektrode aufnimmt, und die zweite Elektrode die Form eines Kreisrings annimmt und die erste Elektrode umgibt.

2. Vorrichtung für kreisförmige Hauttiefenbehandlung nach Anspruch 1, weiter umfassend eine Vakuumpumpe.

3. Vorrichtung für kreisförmige Hauttiefenbehandlung nach Anspruch 1, **dadurch gekennzeichnet, dass** das Handstück der Vorrichtung aus einem Griff (1), einem Träger (2) besteht, dass der Griff (1) ergonomisch geformt ist und eine Nut (1a) im Zentrum entlang seiner Länge aufweist, die in einer kurzen zylindrischen Nut (1b) in der Nähe des Trägers (2) endet, dass der Griff (1) Gewinde an einem Abschnitt (1c) aufweist, der zum Verschrauben in den Träger (2) beabsichtigt ist, dass der Träger (2) kuppelförmig ist und in seinem Inneren eine viertelkreisförmige Nut (2a) und eine enge Nut (2b) aufweist, die in einer Bohrung (2c) endet, in der eine Abdeckung (2d) positioniert ist, dass sich während des Betriebs der Vorrichtung mit Vakuum die Abdeckung (2d) in engem Kontakt mit dem Träger (2) befindet, dass der untere Teil des Trägers (2) mit Gewinden versehen ist, die dazu dienen, den glockenförmigen Träger (3) zu verschrauben, nachdem er in dem zylindrischen Träger (4) positioniert wurde, dass der zylindrische Träger (4) eine breite Vertikalnut (4a) im Zentrum und horizontale Nuten (4b und 4c) aufweist, dass eine Vertikalnut (4d) von der unteren Linie von Nuten (4b und 4c) in dem Zentrum des Trägers (4) verläuft, dass die erste Elektrode (5) des Kondensators zusammen mit dem dielektrischen Gehäuse (5a) an der Unterseite des Trägers (4) mittels eines Elementes (7) befestigt ist, dass der glockenförmige Träger (3) mit einer Öffnung im Zentrum versehen ist, die zum Befestigen in einen festen Bestandteil mit dem Träger (2) und zylindrischen Träger (4) beabsichtigt ist, dass sich von dem horizontalen Abschnitt des glockenförmigen Trägers (3) die Seite gleichmäßig abwärts zu ca. 1/6 von der Höhe erstreckt und einen halbkreisförmigen Kragen (3a) bildet, in den die ringförmige Elektrode (6) des Kondensators angeordnet ist, dass die Elektrode (6) in einer Weise gebogen ist, dass sie sich der Kurve des Kragens (3a) in dem Abschnitt des Trägers (3) anpasst, der sich in Verwendung am nächsten an der Haut des Patienten befindet, dass der Kragen (3a) durch eine ringförmige Abdeckung (3b) geschlossen ist, dass ein Luftansaugrohr (8), ein Draht (9) für die Versorgung der ersten Elektrode (5) und ein Draht (10) für die Versorgung der zweiten Elektrode (6) durch die Nut (1a) des Handstücks (1) angeordnet sind, dass der Draht (9) ebenfalls durch die kreisförmig geformte Nut (2a) von dem Träger (2) und die Vertikalnut (4d) von dem zylindrischen Träger (4) eingeführt ist, dass es an dem anderen Ende des Handstücks (1) einen Schlauch (11) gibt, der zu einem Gehäuse der Vorrichtung führt und das sichere Handhaben der Vorrichtung ermöglicht.

4. Vorrichtung für kreisförmige Hauttiefenbehandlung nach Anspruch 1 oder 2, **dadurch gekennzeichnet, dass** die zweite Elektrode (13) eine Form eines horizontalen Kreisrings aufweist.

5. Vorrichtung für kreisförmige Hauttiefenbehandlung nach Anspruch 1 oder 2, **dadurch gekennzeichnet, dass** die zweite Elektrode (6) in einer Weise gebogen ist, sodass sie sich der Kurve des Kragens (3a) im Abschnitt des Trägers (3) anpasst, der sich beim Verwenden am nächsten an der Haut des Patienten befindet.

6. Vorrichtung für kreisförmige Hauttiefenbehandlung nach einem der Ansprüche 1 bis 5, **dadurch gekennzeichnet, dass** die Elektroden (5, 6, 12, 13) elektrisch gekoppelt sind, um eine Elektrode zu bilden, wobei eine weitere neutrale Elektrode in der Form einer rechteckigen oder runden Platte angepasst ist, um an einer entfernten Körperpartie angeordnet und mit dem Erzeuger des HF-Signals verbunden zu werden.

7. Vorrichtung für kreisförmige Hauttiefenbehandlung nach den Ansprüchen 1 bis 6, **dadurch gekennzeichnet, dass** eine weitere getrennte dritte Elektrode in das Handstück integriert ist.

8. Vorrichtung für kreisförmige Hauttiefenbehandlung nach den Ansprüchen 1 bis 7, **dadurch gekennzeichnet, dass** eine oder mehrere Ultraschallquellen über der ersten Elektrode eingesetzt sind, die auf einer mechanischen und/oder thermischen Basis auf das Gewebe wirken.

9. Vorrichtung für kreisförmige Hauttiefenbehandlung nach den Ansprüchen 1 bis 7, **dadurch gekennzeichnet, dass** eine Ultraschallquelle über der ersten Elektrode eingesetzt ist, die aus einem piezoelektrischen Kristall hergestellt ist.

10. Vorrichtung für kreisförmige Hauttiefenbehandlung nach den Ansprüchen 1 bis 7, **dadurch gekennzeichnet, dass** eine oder mehrere Quellen von sichtbarem und/oder Infrarotlicht in das Handstück der Vorrichtung eingesetzt sind.

11. Vorrichtung für kreisförmige Hauttiefenbehandlung nach den Ansprüchen 1 bis 7, **dadurch gekennzeichnet, dass** zwei, drei oder noch mehr voneinander getrennte und ineinander angeordnete Elektroden eines Kondensators in das Handstück der Vorrichtung eingesetzt sind.

12. Vorrichtung für kreisförmige Hauttiefenbehandlung nach den Ansprüchen 1 bis 11, **dadurch gekennzeichnet, dass** die erste Elektrode des Kondensators eine ovale Form aufweist und von der zweiten Elektrode umgeben ist, die eine Form eines ovalen Rings aufweist.

## Revendications

1. Dispositif de thérapie profonde circulaire de la peau par application capacitive de champs électriques de radiofréquences, ledit dispositif comprenant une pièce à main d'application, une alimentation électrique et un générateur d'un signal de radiofréquences, la pièce à main du dispositif logeant une première (5, 12) et une deuxième (6, 13) électrodes isolées électriquement d'un condensateur, dans lequel ladite première électrode (5, 12) du condensateur est de forme circulaire, est logée à l'intérieur d'un logement diélectrique (5a) et est agencée au fond d'un support cylindrique (4) encerclé par un support en forme de cloche (3) ayant un collier (3a) logeant ladite deuxième électrode, ladite deuxième électrode prenant la forme d'un anneau circulaire et encerclant ladite première électrode.

2. Dispositif de thérapie profonde circulaire de la peau selon la revendication 1, comprenant en outre une pompe à vide.

3. Dispositif de thérapie profonde circulaire de la peau selon la revendication 1, **caractérisé en ce que** ladite pièce à main dudit dispositif se compose d'une poignée (1) et d'un support (2), **en ce que** ladite poignée (1) est de forme ergonomique et comporte une rainure (1a) au centre dans le sens de sa longueur qui se termine dans une rainure cylindrique courte (1b) à proximité dudit support (2), **en ce que** ladite poignée (1) comporte des filetages filetés sur une partie (1c) destinée au vissage dans ledit support (2), **en ce que** ledit support (2) est en forme de coupole et comporte, à l'intérieur de celui-ci, une rainure en forme de quart de cercle (2a) et une rainure étroite (2b) se terminant dans un trou perforé (2c), dans lequel un couvercle (2d) est positionné, **en ce que**, pendant le fonctionnement du dispositif sous vide, ledit couvercle (2d) est en contact étroit avec ledit support (2), **en ce que** la partie inférieure dudit support (2) est pourvue de filetages servant à visser ledit support en forme de cloche (3), après avoir été positionné, dans ledit support cylindrique (4), **en ce que** ledit support cylindrique (4) comporte une rainure verticale large (4a) dans les rainures centrales et horizontales (4b et 4c), **en ce qu'**une rainure verticale (4d) s'étend à partir de la ligne inférieure des rainures (4b et 4c) dans le centre dudit support (4), **en ce que** ladite première électrode (5) du condensateur est attachée au dit logement diélectrique (5a) au fond dudit support (4) au moyen d'un organe (7), **en ce que** ledit support en forme de cloche (3) est pourvu d'un trou au centre destiné à un attachement à une partie intégrante dudit support (2) et du support cylindrique (4), **en ce que**, à partir de la partie horizontale dudit support en forme de cloche (3), le côté s'étend uniformément vers le bas à environ 1/6 de la hauteur et forme un collier semi-circulaire (3a), dans lequel ladite électrode en forme d'anneau (6) du condensateur est agencée, **en ce que** ladite électrode (6) est pliée de manière à s'adapter à la courbe dudit collier (3a) dans la partie dudit support (3) qui, en utilisation, est la plus proche de la peau du patient, **en ce que** ledit collier (3a) est fermé par un couvercle en forme d'anneau (3b), **en ce qu'**un tube d'aspiration d'air (8), un fil (9) pour l'alimentation de ladite première électrode (5) et un fil (10) pour l'alimentation de ladite deuxième électrode (6) sont agencés à travers ladite rainure (1a) de ladite pièce à main (1), **en ce que** ledit fil (9) est également introduit à travers ladite rainure de forme circulaire (2a) dudit support (2) et ladite rainure verticale (4d) dudit support cylindrique (4), **en ce que**, à l'autre extrémité de ladite pièce à main (1), il y a un tube flexible (11) conduisant à un logement dudit dispositif et permettant une manipulation en toute sécurité dudit dispositif.

4. Dispositif de thérapie profonde circulaire de la peau selon la revendication 1 ou 2, **caractérisé en ce que** ladite deuxième électrode (13) a une forme d'un anneau circulaire horizontal.

5. Dispositif de thérapie profonde circulaire de la peau selon la revendication 1 ou 2, **caractérisé en ce que** ladite deuxième électrode (6) est pliée de manière à s'adapter à la courbe dudit collier (3a) dans la partie dudit support (3) qui, en utilisation, est la plus proche de la peau du patient.

6. Dispositif de thérapie profonde circulaire de la peau selon l'une quelconque des revendications 1 à 5, **caractérisé en ce que** lesdites électrodes (5, 6, 12, 13) sont électriquement couplées pour former une électrode, dans lequel une autre électrode neutre ayant la forme d'une plaque rectangulaire ou ronde est apte à être agencée sur une partie distante du corps et reliée au générateur de signal de radiofréquences.

7. Dispositif de thérapie profonde circulaire de la peau selon l'une quelconque des revendications 1 à 6, **caractérisé en ce qu'**une troisième électrode davantage séparée est intégrée dans ladite pièce à main.

8. Dispositif de thérapie profonde circulaire de la peau selon l'une quelconque des revendications 1 à 7, **caractérisé en ce qu'**une ou plusieurs sources d'ultrasons sont insérées au-dessus de la première électrode agissant sur le tissu sur une base mécanique et/ou thermique.

9. Dispositif de thérapie profonde circulaire de la peau selon l'une quelconque des revendications 1 à 7, **caractérisé en ce qu'**une source d'ultrasons est insérée au-dessus de ladite première électrode constituée d'un cristal piézo-électrique.

10. Dispositif de thérapie profonde circulaire de la peau selon l'une quelconque des revendications 1 à 7, **caractérisé en ce qu'**une ou plusieurs sources de lumière visible et/ou infrarouge sont insérées dans ladite pièce à main du dispositif.

11. Dispositif de thérapie profonde circulaire de la peau selon l'une quelconque des revendications 1 à 7, **caractérisé en ce que** deux, trois ou plus d'électrodes mutuellement séparées et agencées l'une dans l'autre d'un condensateur sont insérées dans ladite pièce à main du dispositif.

12. Dispositif de thérapie profonde circulaire de la peau selon l'une quelconque des revendications 1 à 11, **caractérisé en ce que** ladite première électrode du condensateur est de forme ovale et encerclée par ladite deuxième électrode ayant la forme d'un anneau ovale.
